Europäisches Patentamt

European Patent Office

Office européen des brevets

(11) Numéro de publication: **0 321 373**
**A1**

## DEMANDE DE BREVET EUROPEEN

(21) Numéro de dépôt: **88440024.3**

(22) Date de dépôt: **23.03.88**

(51) Int. Cl.⁴: **B 60 K 28/00**

(30) Priorité: **16.12.87 FR 8717788**

(43) Date de publication de la demande:
**21.06.89 Bulletin 89/25**

(84) Etats contractants désignés:
**AT BE CH DE ES GB GR IT LI LU NL SE**

(71) Demandeur: **Dana, Dominique**
**Le Mas du Cade Quartier des Plaines**
**F-83440 Tanneron (FR)**

(72) Inventeur: **Dana, Dominique**
**Le Mas du Cade Quartier des Plaines**
**F-83440 Tanneron (FR)**

(74) Mandataire: **Bossard, Jacques-René**
**Cabinet MEYER & COURTASSOL Bureau EUROPE 20**
**Place des Halles**
**F-67000 Strasbourg (FR)**

(54) Dispositif alcootest intégrable à un véhicule.

(57) Dispositif alcootest intégrable dans un véhicule automobile, caractérisé en ce qu'un système d'alcootest classique (1) est relié à un montage électronique avec vu-mètre à diodes électroluminescentes (8) affichant le résultat du test effectué par un comparateur (7), l'alimentation du système étant réalisée par connexion au circuit électrique du moteur.

**Description**

## DISPOSITIF ALCOOTEST INTEGRABLE A UN VEHICULE.

La présente invention concerne un dispositif d'"alcootest" intégrable dans tout véhicule automobile et agissant sur le fonctionnement dudit véhicule après traitement de l'information issue du test.

Les alcootest sont des appareils bien connus et répandus par exemple dans la police de la route. Il s'agit d'un appareil dont le modèle le plus courant consiste en un tube de verre rempli d'un réactif tel que l'oxyde de chrome. A l'une des extrémité est fixé un embout par lequel le sujet souffle, tandis qu'à l'autre extrémité se trouve un sac en plastique de volume défini. Le réactif vire de couleur sur une longueur d'autant plus grande que l'haleine du sujet est chargée d'alcool : on mesure en fait la teneur en alcool contenue dans l'air expiré.

Le domaine dans lequel l'alcootest est le plus utilisé est celui de la conduite automobile. Cela se conçoit lorsqu'on mesure le danger que peut constituer un chauffeur dont le taux d'alcool dans le sang est excessif, avec les effets que l'on sait. Cependant, ce test n'est pas systématiquement effectué et ce qui est plus grave, c'est bien souvent à postériori qu'on constate la surconsommation d'alcool. L'accident est alors arrivé, ou en est réduit à essayer de guérir, réparer faut d'avoir pu prévenir.

Quelle que soit la quantité d'alcool absorbée, le buveur peut emprunter son véhicule pour effectuer le trajet qu'il veut. Il n'y a pas de contrôle de son état objectif. Ses facultés et réflexes largement diminués lui enlèvent pourtant une grande partie de sa lucidité dans la prise de risque.

La conséquence directe est que le nombre d'accidents de la route imputable à l'alcool est élevé. C'est pourquoi, on se préoccupe de ce problème dont la gravité va croissante. Ainsi, outre des campagnes de sensibilisation, on assiste à une modification de la législation et à un changement de jurisprudence dans le sens d'une plus grande sévérité. La question est de savoir si de telles mesures auront un impact sensible face à des habitudes solidement ancrées.

Le dispositif suivant l'invention permet de réaliser une action effective et systématique dans les véhicules qui en sont équipés. Il s'agit d'un boîtier de taille réduite comprenant un alcootest de type classique ainsi qu'un montage électronique permettant le traitement de l'information, le tout étant relié à une source d'énergie électrique, qui n'est autre que celle du véhicule.

Selon l'invention, on dispose de trois variantes :
- deux modèles non coercitifs, n'ayant aucune action directe sur le moteur et qui se contentent de fournir une indication sur le taux d'alcool dans le sang.
- préférentiellement un modèle coercitif qui est raccordé directement au circuit du démarreur du moteur et qui empêche le démarrage si le dispositif ne l'autorise pas à la suite du test.

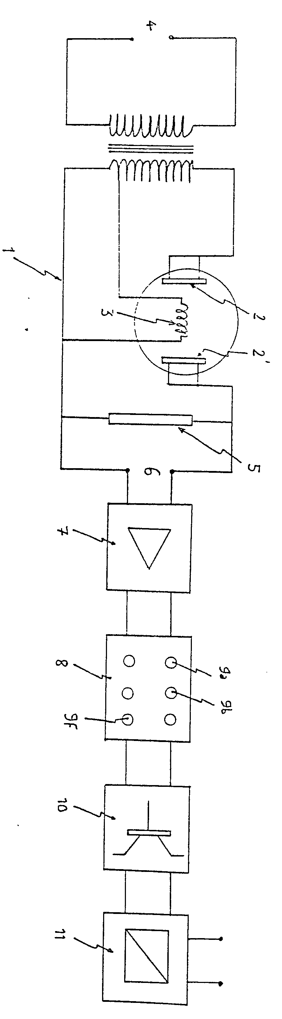

La figure unique du dessin annexé illustre schématiquement le mode de réalisation le plus sophistiqué de l'invention, c'est-à-dire celui dans lequel la sortie de l'alcootest exerce une action coercitive en interdisant le fonctionnement du démarreur de la voiture quand la teneur en vapeur d'éthanol de l'haleine du conducteur dépasse une valeur prédéterminée.

Selon ce mode de réalisation, le système se compose d'un capteur ou détecteur de vapeurs d'éthanol, 1, qui peut être de type connu, tel que par exemple celui commercialisé sous la dénomination TGS 812 par la firme japonaise FIGARO ENGINEERING INC d'Osaka. Un tel détecteur comporte un couple d'électrodes sensibles 2-2', constituée chacune par une masse de matériau semi-conducteur fritté (Bioxyde d'étain), échauffées par une résistance 3 constituée par un enroulement en fil de chrome. Ces électrodes sont alimentées en courant électrique à partir de la source 4, et la variation de leur résistance 5 en fonction de la concentration en gaz présent est mesurée par la variation de tension de sortie en 6 du système. Quand la concentration en gaz augmente, l'impédance du capteur diminue, de sorte que la tension de sortie augmente. Ces variations de tension sont, selon l'invention, comparées dans le comparateur 7 à des valeurs étalons, qui déclenchent, en 8, l'une ou l'autre d'une série de diodes électroluminescentes (LED) 9a, 9b....9f, de manière à ce que chaque diode allumée remplisse une fonction d'affichage, en maintenant sa valeur maximale mesurée, même lorsque le capteur ne reçoit plus de vapeurs, jusqu'à sa remise à zéro.

Selon l'invention, l'allumage d'une diode prédéterminée 9k, correspondant à la tension de sortie maximale admissible déterminée par le comparateur 7, déclenche à son tour un circuit de commande 10 dont la fonction est d'interdire la mise en marche du démarreur 11, jusqu'a remise à zéro du système.

Le fonctionnement du dispositif selon l'invention est donc le suivant :

Le démarrage est impossible si l'usager n'est pas soumis au test, le circuit du démarreur étant alors coupé. Si la teneur en alcool dans l'air expiré est excessive, le circuit est également inhibé. La condition nécessaire et suffisante pour que le véhicule soit utilisable est par conséquent que le chauffeur se soit soumis à un essai concluant.

L'installation d'un tel dispositif est possible sur tous les véhicules automobiles, le circuit électrique étant modifié en conséquence, de façon mineure. Par la suite, lorsque l'appareil est installé, on ne peut le désactiver par simple déconnexion, ce qui nuirait à l'efficacité du système.

Comme indiqué précédemment, selon des variantes moins préférentielles, le dispositif alcootest n'a pas d'action directe sur le moteur du véhicule.

Il peut s'agir d'un appareil directement monté par le constructeur dans toutes les voitures sortant d'usine et qui a pour but de donner une indication à l'utilisateur. Selon le résultat, le sujet prend la décision de prendre son véhicule ou non.

Enfin, une autre variante consiste à fournir un dispositif également non coercitif disponible dans le commerce et qui est facilement intégrable dans le véhicule, par exemple en se branchant sur la prise allume-cigare.

Bien entendu, les dispositifs ainsi décrits ne sont donnés qu'à titre d'illustration et les exemples de mise en oeuvre ne sont en aucun cas limitatifs.

## Revendications

1. Dispositif alcootest intégrable dans un véhicule automobile, caractérisé en ce qu'un système d'alcootest classique est relié à un montage électronique avec vu-mètre à diodes électroluminescentes affichant le résultat du test effectué par un comparateur, l'alimentation du système étant réalisée par connexion au circuit électrique du moteur.

2. Dispositif alcootest selon la revendication 1, caractérisé en ce qu'il inhibe le démarreur, quand la teneur détectée en vapeurs d'étanol dépasse une valeur prédéterminée.

3. Dispositif alcootest selon les revendications 1 et 2, caractérisé en ce que sa mise en fonction suivie de l'exécution du test constituent les conditions nécessaire au démarrage ultérieur du moteur, par action sur le démarreur, le fait que le résultat du test soit négatif en étant la condition suffisante.

4. Dispositif alcootest suivant la revendication 1, caractérisé en ce qu'il fait partie intégrante du véhicule, mais n'a pas d'action sur le fonctionnement du moteur.

5. Dispositif alcootest selon les revendications 1 et 2, caractérisé en ce qu'il est indépendant du véhicule et peut être raccordé à la source d'énergie du moteur par le biais de la prise allume-cigare.

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | DE-A-3 403 450 (SIMON) <br> * Page 8, ligne 16 - page 9, ligne 15; page 10, lignes 1-15,28-35; page 13, ligne 15 - page 15, ligne 14; figures 1,2 * | 1-3 | B 60 K 28/00 |
| Y | --- | 4,5 | |
| Y | US-A-4 039 852 (MIYAMOTO et al.) <br> * Colonne 1, lignes 1-12; colonne 3, ligne 62 - colonne 4, ligne 9, lignes 32-65; colonne 5, lignes 33-68; colonne 10, ligne 12 - colonne 12, ligne 50; colonne 14, lignes 39-48; figures 1-3 * | 4 | |
| A | --- | 1-3 | |
| Y | US-A-3 953 831 (ESTRADA) <br> * Colonne 1, ligne 67 - colonne 2, ligne 7; colonne 2, lignes 24-63; colonne 3, lignes 2-16; figure 1 * | 5 | |
| A | DE-A-2 218 166 (BORG-WARNER) <br> * Page 1, lignes 3-5; page 4, lignes 20-23; page 13, lignes 1-7; page 14, ligne 1 - page 18, ligne 8; page 38, lignes 14-19; figures 5,6a,6b * <br> ----- | 1-3 | **DOMAINES TECHNIQUES RECHERCHES (Int. Cl.4)** <br><br> B 60 K <br> A 61 B |

Le présent rapport a été établi pour toutes les revendications

| Lieu de la recherche | Date d'achèvement de la recherche | Examinateur |
|---|---|---|
| LA HAYE | 10-08-1988 | CLASEN M.P. |